# EUROPEAN PATENT SPECIFICATION

(11) **EP 0 344 177 B1**
(45) Date of publication and mention of the grant of the patent: **11.05.1994**
(21) Application number: 88900994.0
(22) Date of filing: 30.12.1987
(51) Int. Cl.: C12M 1/00, B01J 8/02

(54) **POROUS WAFER FOR SEGMENTED SYNTHESIS OF BIOPOLYMERS**
PORÖSER WAFER ZUR SEGMENTIERTEN HERSTELLUNG VON BIPOLYMEREN
PLAQUETTE GAUFREE POREUSE POUR LA SYNTHESE SEGMENTEE DE BIOPOLYMERES

(30) Priority: 06.01.1987 US 716
(43) Date of publication of application: 06.12.1989
(73) Proprietor: BAYLOR COLLEGE OF MEDICINE, Houston, TX 77030 (US)
(72) Inventor: BEATTIE, Kenneth, L., Houston, TX 77096 (US); FROST, James, D., III, Houston, TX 77005 (US)
(74) Representative: Henkel, Feiler, Hänzel & Partner
(86) International application number: US8703462
(87) International publication number: WO8805074

(56) References cited:
- EP-A- 0 164 206
- US-A- 3 401 139
- US-A- 3 647 390
- US-A- 3 763 879
- US-A- 4 155 846
- US-A- 4 678 769

## Description

### BACKGROUND OF THE INVENTION

The present invention relates to the chemical synthesis of biopolymers, and specifically, to a device for the simultaneous synthesis of large numbers of biopolymers, for example, polynucleotides, polypeptides and polysaccharides.

The development of methods for the chemical synthesis of biopolymers of any desired sequence has resulted in great advances in many areas of biology and medicine during recent years. For example, physical and biochemical studies of the structure and interactions of synthetic DNA fragments has led to important new findings concerning the molecular mechanisms of genetic processes, including DNA metabolism, and regulation of gene expression. Synthetic polynucleotides have played a key role in studies of genetic organization through their use as primers for DNA sequencing and as hybridization probes, linkers and adapters in the cloning of genes. An additional use of synthetic polynucleotides is in DNA probe technology in the diagnosis of disease. Ultimately, synthetic polynucleotides may be used in gene replacement therapy to cure genetic disorders, and in other genome engineering procedures to provide resistance to disease and starvation. Synthetic polynucleotides are routinely used for site-directed in vitro mutagenesis, for studying the structure-function relationships within genetic regulatory elements and for determining the effects of specific amino acid substitutions on the functions of proteins. The latter use, termed protein engineering, will not only facilitate an understanding of the mechanism of action of enzymes and other proteins, but will also permit the design of functionally superior proteins and drugs, leading to important advancements in medicine, agriculture and industry. Likewise, the availability of synthetic defined-sequence polypeptides is bringing about equally dramatic advancements in protein chemistry, immunology, pharmacology and biotechnology.

In many genetic engineering projects it is necessary to use a large number of different defined sequence polynucleotides, sometimes hundreds of different sequences in a single experiment. Similarly, some protein chemistry experiments require hundreds of different peptide sequences. In order to determine the nucleotide sequence of the human genome (a project soon to be initiated, with involvement of many laboratories), on the order of fifty million different polynucleotide primers will be required. The latter endeavor, along with many other worthwhile projects that could be carried out by individual laboratories, are economically impractical with the current cost to the investigator of synthetic polynucleotides ($5-$20 per nucleotide residue).

The capability to chemically synthesize polynucleotides of defined sequence resulted from the pioneering work of Michelson and Todd in the 1950s, (Michelson, A.M. & Todd, Sir A.R., "Nucleotides Part XXXII. Synthesis of a Dithymidine Dinucleotide Containing a 3':5' Internucleotide Linkage," J. Chem. Soc. 1955, pp. 2632-2638), in which a method was developed for specific chemical synthesis of 5'-3' internucleo-tide phosphodiester linkages. This procedure was developed further over the next 20 years, culminating in the total synthesis of a gene for transfer RNA by Khorana and Associates, (Khorana, H.G., "Total Synthesis of a Gene," Science, Vol. 203, pp. 614-625, (1979). Recently, the phosphate diester method has been replaced by the phosphate triester method (Letsinger, R.L. and Ogilvie, K.K., "A Convenient Method for Stepwise Synthesis of Oligothymidylate Derivatives in Large-Scale Quantities," J. Am. Chem. Soc., Vol. 89, pp. 4801-4803, (1967); Narong, S.A., Brousseau, R., Hsiung, H.M. and Michniewicz, J.J., "Chemical Synthesis of Deoxyoligonucleotides by the Modified Triester Method, Meth. Enzymol, Vol. 65, pp. 610-620, (1980)) and the phosphite triester method (Letsinger, R.L., Finnan, J.L., Heavener, G.A. and Lunsford, W.B., "Phosphite Coupling Procedure for Generating Internucleotide Links," J. Am. Chem. Soc., Vol. 97, pp. 3278-3279, (1975); Beaucage, S.L. and Caruthers, M.H., "Deoxynucleotide Phosphoramidites - A New Class of Key Intermediates For Deoxypolynucleotide Synthesis," Tet. Lett., Vol. 22, pp. 1859-1862, (1981)), which have the advantage of more rapid synthesis and fewer side reactions. Both of these methods can be carried out in solution as originally devised, but have recently been adapted for solid phase synthesis of polynucleotides (Matteucci, M.D. and Caruthers, M.H., "Synthesis of Deoxyoligonucleotides on a Polymer Support," J. Am. Chem. Soc., Vol. 103, pp. 3185-3191, (1981); Sproat, B.S. and Bannwarth, W., "Improved Synthesis of Oligodeoxynucleotides On Controlled Pore Glass Using Phosphotriester Chemistry and a Flow System," Tet. Lett., Vol. 24, pp. 5771-5774, (1983)). Solid phase synthesis offers the advantage of greater speed of synthesis because the growing chain is covalently attached to an insoluble support, permitting reagents to be washed away between chemical steps and obviating the need to purify the polynucleotide product after each addition of monomer. Furthermore, solid phase synthesis permits automation of the process, so that each base addition (via multistep reaction cycle) can be carried out in about ten minutes at ambient temperature. The high efficiency of condensation under these conditions (currently >99%) permits the automated synthesis of polydeoxynucleotides of chain length greater than 100.

Chemical procedures used for solid phase synthesis of polypeptides are frequently based on the original protocol of Merrifield, which was successfully used for synthesis of enzymically active, 124-residue ribonuclease A (Gutte, B. and Merrifield, R.B., "The Synthesis of Ribonuclease A," J. Biol. Chem., Vol. 246, pp. 1922-1941, (1971)). This procedure uses standard polystyrene-divinylbenzene supports, t-butyloxy-carbonyl (Boc) amino group protection, and DCC-activated condensation with symmetric anhydride intermediates. The procedure has been used successfully in automated peptide synthesizers, as well as in the multiple simultaneous synthesis methods of Houghton or Kubodera et al. below.

Several alternative procedures for peptide synthesis have been devised. One particularly advantageous one (Auffret, A.D. and Meade, L.G., "Alternative Strategies in Peptide Synthesis," Synthetic Peptides in Biology and Medicine, Alitalo, K., Partanen, P. and Vaheri, A. (Eds..), Elsevier Science Publishers, Amsterdam, 1985) utilizes a composite polyamide-Kieselguhr support (found to be superior for continuous flow synthesis), fluorenylemethoxycarbonyl (Fmoc) amino group protection, and 1-hydroxybenzatriazole-activated condensation with pentafluorophenyl ester (PFPE) intermediates. The high stability of the active ester intermediates make them more conveniently used for peptide synthesis than the relatively unstable anhydride intermediates.

Recent developments in polynucleotide synthesis, including descriptions of the chemical reactions, are summarized in review articles by John Smith ("Automated Solid Phase Oligodeoxyribonucleotide Synthesis", American Biotechnology Laboratory, pp. 15-24 (December 1983)) and Marvin Caruthers ("Gene Synthesis Machines: DNA Chemistry and Its Uses", Science, Vol. 230, pp. 281-85 (1985)). One particularly promising recent advancement is the development of cost-effective procedures for in situ generation of phosphoramidite intermediates from inexpensive protected nucleosides (Barone, A.D., Tang, J.-Y. and Caruthers, M.H., "In Situ Activation of Bis-Dialkylaminophosphines - A New Method for Synthesizing Deoxyoligonucleotides on Polymer Supports," Nucl. Acids Res., Vol. 12, pp. 4051-4061, (1984); Nielsen, J., Taagaard, M., Marigg, J.E., van Boom, J.H. and Dahl, O., "Application of 2-cyanoethyl N, N, N', N' - tetraisopropylphosphorodiamidite for In Situ Preparation of Deoxyribonucleoside Phosphoramidites and Their Use in Polymer - Supported Synthesis of Oligodeoxyri-bonucleotides," Nucl. Acids Res., Vol. 14, pp. 7391-7403, (1986)).

Another advantageous recent development is the use of amidine groups to protect exocyclic amino groups (e.g., Caruthers, M.H., McBride, L.J., Bracco, L.P. and Dubendorff, J.W., "Studies on Nucleotide Chemistry 15. Synthesis of Oligodeoxynucleotides Using Amidine Protected Nucleosides," Nucleosides and Nucleotides, Vol. 4, pp. 95-105, (1985)). Amidine protecting groups stabilize deoxyadenosine residues against acid-catalyzed depurination, which occurs during the detritylation step of the synthesis cycle, thereby permitting synthesis of longer polynucleotides.

Finally, a procedure for synthesis of RNA polymers on silica supports, involving a modified phosphoramidite approach, has recently been reported (Kierzek, R., Caruthers, M.H., Longfellow, C.E., Swinton, D., Turner, D.H. and Freier, S.M.," Polymer-Supported RNA Synthesis and its Application to Test the Nearest - Neighbor Model for Duplex Stability," Biochemistry, Vol. 25, pp. 7840-7846, (1986)).

Although the above methods permit the synthesis of one or a few polynucleotide sequences at a time, at moderate cost, there is a great need for technological development in this area, to reduce the cost of synthesis and to permit simultaneous synthesis of large numbers of polynucleotide sequences. Progress toward this aim has recently been made in the form of procedures and devices that permit multiple simultaneous synthesis of polynucleotides or polypeptides.

Frank et al. ("A New General Approach for the Simultaneous Chemical Synthesis of Large Numbers of Oligonucleotides: Segmented Solid Supports", Nucleic Acid Research, Vol. 11, No. 13, pp. 4365-77 (1983)) recently used cellulose filters as a solid phase support for polynucleotide synthesis. A protected nucleoside was covalently linked to the hydroxyl groups of the filter paper by 3'-o-succinate linkage, then elongated by the phosphate triester procedure used previously with loose beaded solid phase support materials. In this paper the authors reported synthesis of two octamers, and proposed that by stacking the paper filters into four different reaction columns, designated for addition of A, G, C and T residues to the growing chain and sorting the discs between reaction cycles, a large number of different polynucleotide sequences could be simultaneously synthesized. The authors demonstrated that the two octamers synthesized by this procedure (present within the same reaction column during most cycles) were obtained at reasonable yield, and DNA sequence analysis proved that the products consisted of the expected nucleoside sequences and were not contaminated by each other.

The proposed use of the filter paper method for simultaneous synthesis of many sequences was later implemented by Matthes et al. ("Simultaneous Rapid Chemical Synthesis of Over One Hundred Polynucleotides on a Microscale", The EMBO Journal, Vol. 3, No. 4, pp. 801-05 (1984)). These authors used a phosphate triester procedure similar to that reported by Frank et al., to simultaneously synthesize over one hundred polynucleotide sequences within a period of two weeks. Several limitations of the Matthes et al. procedure exist. Due to low loading capacity of the filter paper disks and their unfavorable mass transfer properties (resulting in less than optimal access of reagents to the growing chain), the coupling efficiency at each step is poor compared with that attained with the standard solid phase synthesis procedures, and only a very small quantity of desired polynucleotide is produced, of limited chain length (up to about 20-mer). The product is heavily contaminated by shorter failure sequences, and must be purified by time-consuming procedures before use. Nevertheless, this procedure has the potential of yielding large numbers of sequences at low cost. This method apparently has been attempted by many laboratories, but apparently only a a very few laboratories have been able to obtain usable products using the technique.

A very recent report (Bannwarth, W. and Laiza, P., "A System for the Simultaneous Chemical Synthesis of Different DNA Fragments on Solid Support," DNA, Vol. 5, pp. 413-419, (1986)) describes a mechanical apparatus that can simultaneously synthesize several different polynucleotides. The device consists of a series of stackable rotatable metal disks, each containing, in radially symmetrical position, a single reaction chamber plus a number of narrow "bypass" holes. The stacked metal disks can be rotated to produce vertical alignment of all reaction chambers designated for addition of a given nucleoside residue to the support-bound DNA chains contained therein, with these chambers being connected by bypass holes. Thus, by appropriate rotation of the metal disks following each reaction cycle (created by sequential flow of reagents and solvents through the system), a different DNA sequence is synthesized for each of the stacked metal disks. The chief advantage of this device over the segmented filter paper method is higher coupling efficiency, enabled by the placement of controlled pore glass supports within the reaction chambers. DNA chains of up to 36 residues long were produced utilizing phosphoramidite chemistry. Another advantage of the design is its potential for automation. The chief disadvantage is the relatively low number of simultaneous synthesis (a maximum of 12 DNA fragments were simultaneously produced).

A similar system, also based on the rotatable element technique, comprises stacked plates each with a reaction chamber and bypass channels (Cole, EP-A-164,206; Hamill, US-A-4-728,502). This system has features similar to the previous system (Bannwarth & Laiza). Both systems are not readily adaptable to peptide synthesis, and both systems have reduced reagent economy due to inherent dead volumes of the bypass channels.

Kubodera, et al. (US-A-3 647,390) teach an apparatus using the chemical procedures described above for solid phase synthesis of polypeptides. This apparatus has the possibility of being automated. However, it is only capable of a single synthesis at a time, and is not readily, if at all, adaptable to high multiple simultaneous synthesis.

In another approach, utilized for simultaneous synthesis of different polypeptides, (Houghton, "General Method for the Rapid Solid-Phase synthesis of Large Numbers of Peptides: specificity of Antigen-Antibody Interaction of the Level of Individual Amino Acids", Proc. Natl. Acad. Sci. USA, v82, pp. 5131-35 (August 1985)), Houghton employed polypropylene mesh bags containing solid phase support resins used for standard solid phase synthesis of peptides. By placing a number of these resin-containing bags into a single stirred reaction chamber, all peptide sequences to which a given amino acid was to be added could undergo the coupling reaction simultaneously. The authors used this procedure to simultaneously synthesize 248 different 13-mer peptides which were obtained in yield comparable to that attained by standard single-peptide solid phase methods. In this work, each of the 13-mer peptides actually consisted of a sequence identical to the "control sequence," except for a single amino acid replacement. Thus, at each amino acid addition, the vast majority of the resin-containing bags were placed into the same stirred reaction vessel, while only those resins containing peptides to which a unique amino acid was to be added at that position in the sequence were reacted separately from the bulk of the material. Although the "different" peptide sequences synthesized in Houghten's original work each consisted of the same sequence, with a single amino acid change from the "control sequence," it was proposed that by use of a multiplicity of stirred reaction vessels, each containing many resin-containing bags, the procedure could be used for simultaneous synthesis of a large number of completely unique peptide sequences. Houghten's "tea bag" method, including description of its use for simultaneous synthesis of 120 entirely different 15-residue peptides, is further described in a recent article (Houghten et al, "Simultaneous Multiple Peptide Synthesis: The Rapid Preparation of Large Numbers of Discrete Peptides for Biological Immunological, and Methodological Studies," Biotechniques, Vol. 4, No. 6, pp. 525-28 (1986)).

Two difficulties may prevent the Houghten "tea bag" method from being implemented for simultaneous synthesis of large numbers of polynucleotide sequences. The sealable polypropylene mesh bags are not sufficiently inert to be used in the phosphate triester and phosphite triester procedures currently used for polynucleotide synthesis. Support-containing porous bags constructed of inert materials such as TEFLON® are difficult, if not impossible to seal, making it difficult to prevent loss of solid phase support from the bags during synthesis. A more serious problem is that in the solid phase procedure for polynucleotide synthesis, sufficient space must be left in the column above the support bed, such that as solvents and reagents are pumped from below, the support is "lifted" by the upward flow, resulting in the necessary mass transfer within the beads required for nearly quantitative chemical reactions. The physical properties of the non-rigid "tea bags" would not permit the necessary lifting of support materials during passage of solvents and reagents through the column.

US-A-4 301 139 discloses a multilayer column chromatography specific binding assay method, a test device and a test kit for use in it. The test device comprises a tube completely filled with different superimposed solid material beds.

US-A-4 155 846 discloses an apparatus comprising a column having a plurality of sequentially arranged segmented chambers filled with adsorptive gel or ion exchange resin for use in chromatography.

US-A-3 763 879 discloses combination columns comprised of individual connective parts, which provide systems for effecting chemical processes such as chromatography and involving carrier-bound reagents.

US-A-3 692 669 discloses an apparatus for micro dry column chromatography, comprising a tubular member filled with a compacted, void-free column of particles of dry chromatographic adsorbent and a porous means at least at one end thereof.

Accordingly, due to the shortcomings of the present devices and procedures, there exists a need for a device and procedure for rapid, simultaneous synthesis of large numbers of any biopolymer of different sequences at high yields and lower costs.

### SUMMARY OF THE INVENTION

It is therefore an object of the present invention to provide an improved device for the chemical synthesis of biopolymers.

Another object of the present invention is to provide an improved device for the simultaneous synthesis of large numbers of biopolymers.

Yet another object of the present invention is to provide for the simultaneous production of large numbers of defined-sequence biopolymers at very low cost.

Another object of the present invention is to provide a device applicable for the simultaneous, solid phase synthesis of any of the various biopolymers.

Still yet another object of the present invention is to provide for the simultaneous production of large numbers of defined-sequence biopolymers at high yields.

Yet an additional object of the present invention is to provide an improved segmented device for simultaneously producing biopolymers.

A further object of the present invention is to provide an improved device for simultaneously producing large numbers of biopolymers requiring lower amounts of reagents and solvents.

Yet a further object of the present invention is to provide an improved device for simultaneously producing large numbers of biopolymers requiring less synthesis time.

A still further object of the present invention is to provide an improved device for simultaneously producing large numbers of biopolymers in which the many segments, hereinafter referred to as "wafers", are easy to separate from one another after each reaction cycle.

An additional object of the present invention is to provide an improved solid phase support segment ("wafer") for the chemical synthesis of biopolymers.

Thus, in accomplishing the foregoing objects, there is provided in accordance with one aspect of the present invention, a segmented wafer synthesis device for the synthesis of multiple defined-sequence biopolymers, comprising a solvent delivery system, at least one column connected to the solvent delivery system to provide solvent and reagent flow through the column, and at least one wafer positioned in the column at which polymeric synthesis occurs. In a preferred embodiment, the synthesis device comprises at least four columns for receiving four reagents, and a plurality of wafers in each column, wherein each of the wafers provides for the synthesis of a defined-sequence polymer. The device can be either automatic, semi-automatic or manual, depending on user needs.

In a further embodiment, the device comprises a plurality of stacked wafers which are connected together to form a column with the solvent delivery system being connected to the column to provide flow through the column.

In accordance with another aspect of the present invention, there is provided a wafer for synthesizing biopolymers, for example, polynucleotides, polypeptides and polysaccharides, comprising a solid phase support material, a retaining ring for retaining the support material in a chamber formed by the inner walls of the retaining ring, and means, for example, a membrane or frit, for allowing flow through the retaining ring to the support material and for preventing migration of the support material from the retaining ring. Preferably, the retaining ring comprises an inner, enclosed reaction chamber for receiving and retaining the support material, the retaining ring being open on both ends. The porous flow means is an essentially inert porous material, and is preferably provided at each end of the retaining ring and extends across the inner chamber to enclose the chamber. In addition, the wafer preferably comprises an inert securing means for securing the support materials to the retaining means.

The solid phase support material advantageously is selected from the group consisting of silica, controlled pore glass (CPG), Polystyrene-divinyl-benzene, polyamide resins, polyamide-Kieselguhr composite resins, macroreticular resins, benzhydrylamine resins, and macroporous plastic resins such as MONOBEADS resin (a resin produced by Pharmacia). The porous support material comprises a derivatized material which includes a covalently attached residue, for example, a nucleoside in the case of polynucleotide synthesis.

The porous membrane or frit preferably comprises flexible membrane composed of TEFLON® or other inert fluorocarbons, or rigid frits of glass, stainless steel or titanium. The porosity of the membrane or frit is sufficiently large to allow flow through the wafer and sufficiently small to retain the porous support material in the wafer.

In one preferred embodiment, the wafer comprises a solid phase support material, an inner housing ring comprising an inner reaction chamber formed by the inner walls of the ring for receiving and retaining the support material, the housing ring being open on both ends, an inert porous membrane or frit positioned at and extending across each of the open ends of the housing ring, the membrane having a larger diameter than the inner ring, and two outer rings having an inner diameter slightly larger than the inner ring, the inner rings encompassing the inner ring and securing the edges of the membrane between the inner ring and the outer rings. Particularly preferred is an outer securing means which comprises a retaining ring positioned about the outer surface of each end of the housing ring.

In another preferred embodiment, the wafer comprises a solid phase support material, an inert cylindrical housing ring, open on both ends, and an inert circular frit snapped into indentations near the open ends of the housing ring.

The wafer design of the present invention provides for the simultaneous production of numerous biopolymers. The geometry of the support material results in high coupling efficiency, and the rigid wafers are easy to sort after each reaction cycle. This arrangement permits the simultaneous synthesis of many different sequences. By using support material of varying capacity (density of derivatization) and by varying the height of each wafer, the scale of synthesis can be varied from less than 0.1 micromole to greater than 10 micromoles per segment. Furthermore, segments of varying heights can be stacked within each column, permitting the simultaneous synthesis of products of widely different scale. The flexibility and efficiency of this approach should permit the synthesis of large numbers of biopolymers at a substantially reduced cost. For example, the present cost of polynucleotide synthesis, under ideal conditions (such as existence of an in-house synthesis service) is typically $10 to $15 per residue. With the segmented wafer device, the cost is significantly less, and possibly as low as $0.50 - $2.00 per residue. Since cost presently remains the limiting factor in the use of synthetic biopolymers, development of the segmented wafer device is another quantum leap in the use of biopolymers in scientific research, and should accelerate future developments in biomedical science.

Further objects, features and advantages will become apparent from a review of the detailed description of the preferred embodiments which follows, in view of the drawings, a brief description of which follows.

### BRIEF DESCRIPTION OF THE DRAWINGS

Referring to the drawings:
Figure 1 is an exploded perspective view of an embodiment of the wafer of the present invention.
Figure 2 is a cross-sectional view of an embodiment of the wafer of the present invention in its assembled state.
Figure 3 is a perspective view of an embodiment of the wafer of the present invention in its assembled state.
Figure 4 is a schematic view of a column assembly of the segmented wafer synthesis device according to the present invention.
Figure 5 is a schematic view of a segmented wafer synthesis device according to the present invention.
Figures 6, 7 and 8 are photographs illustrating the UV shadowing visualization of DNA produced by the present invention.

All numerical references in the figures will be consistent such that the same part in different figures will have the same number.

### DETAILED DESCRIPTION OF PREFERRED EMBODIMENTS

The present invention will first be described by reference to the drawings. At various points in the following disclosure, the present invention is discussed in terms of polynucleotide synthesis. The invention, as has been noted, is equally applicable and useful for the production of any biopolymer that can be synthesized on solid phase supports. Furthermore, the following discussion and drawings primarily describe and illustrate one specific wafer design. It is understood that this description is for illustrative purposes only, and other wafer designs are possible and within the scope of the present invention.

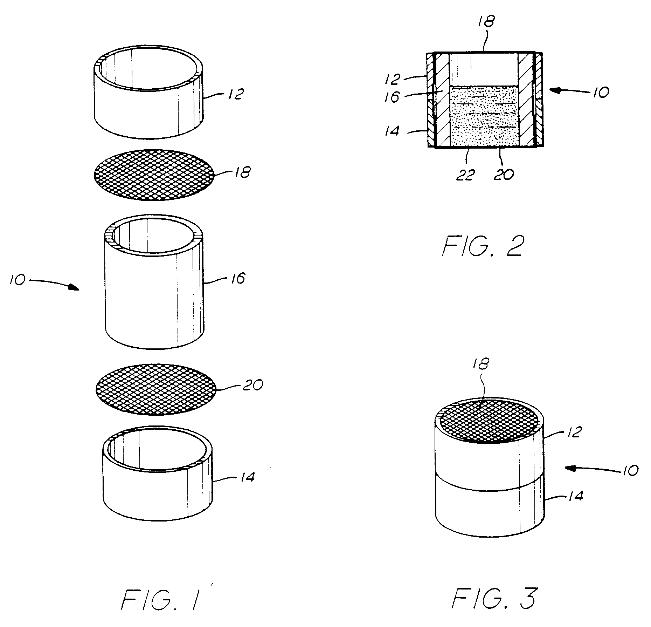

Figure 1 illustrates the wafer 10 of the present invention prior to assembly, i.e., in an exploded schematic view. The wafer comprises an outer securing means comprising upper and lower retaining rings 12 and 14. Located between the opposing retaining rings 12 and 14 is an internal housing ring 16, which together with membranes 18 and 20 serves as the reaction chamber. The wafer further includes porous materials or membranes 18 and 20 positioned at and extending across either end of the housing ring 16 and secured between the housing ring 16 and the retaining rings 12 and 14.

The housing ring 16 has an outer diameter just slightly smaller than the inner diameter of the retaining rings 12 and 14. The porous membranes 18 and 20 have outer diameters greater than the outer diameters of the retaining rings.
It is to be noted that ring, as used here in referring to both the inner housing ring and the retaining rings, includes both circular, rectangular, square and other geometric variations in ring design. The important design criteria is that the rings have a hollow interior space for retaining the reactant components, as described below.

The wafer 10 is shown in its assembled state in Figures 2 and 3. To assemble the wafer, the porous membrane 20 is placed onto the lower retaining ring 14 such that the edges of the membrane extend past the ring around its entire outer circumference. The housing ring 16 is then placed on the lower membrane 20 and pushed into the lower retaining ring 14. The diameters of rings 12, 14 and 16 are selected, with the membrane, to form a fluid tight seal between the rings. After placement of solid phase support material into the housing ring, the upper membrane 18, retaining ring 12 and housing ring 16 are similarly sealed by placing membrane 18 over ring 16 and pushing ring 12 into place. In addition to creation of the fluid tight seal, the design facilitates the retention of the membranes firmly in place during the chemical reaction. This result is achieved by overlapping the edges of the membranes over the housing ring 16 and anchoring the membranes between the retaining rings 12 and 14 and housing ring 16.

As Figures 2 and 3 illustrate, in the assembled wafer 10, the membranes 18 and 20 extend across the ends of the inner housing ring 16, with the ends of the membrane held between the outer retaining rings 12 and 14 and the inner housing ring 16.

The assembled wafer contains the reactant components 22. The reactant components are solid phase supports which have been derivatized by covalently linking a residue, e.g., nucleoside, to the solid support via an organic spacer arm. The residue, or first base, from which polymeric growth will begin, is thus separated from the surface of the support material. The reactant components 22 are placed in the inner housing ring 16 prior to sealing the wafer with membrane 18 and retaining ring 12. Thus, the housing ring 16 and membranes 18 and 20 together form a reaction chamber for the reactant components 22.

As previously noted, the above disclosure is directed to one particular wafer design. It is emphasized that numerous wafer designs are possible and within the scope of the present invention. For example, the wafer could include a snap-together or screw-together design. In particular, an alternative embodiment of wafer 10, could have rigid porous frits, snapped into indentations in the inside surface of the housing ring near its upper and lower edge.

The wafer is a rigid, chemically inert chamber so that it will not interfere with or react with the chemicals used in the synthesis of the biopolymers. The outer retaining rings and inner housing ring can be fabricated from a variety of inert materials, for example, TEFLON® and other fluorocarbons, such as KEVLAR® and KALREZ®.

The size of the wafers can vary over a wide range. For synthesis of milligram quantities of polynucleotides, the inner diameter of the inner retaining ring is preferably in the range of about 2-10 mm, and the height is from about 2-10 mm. For gram quantities of product, the inner diameter is preferably from about 20-100 mm, and the height is from about 20-100 mm. Furthermore, the height of the column of stacked wafers can be increased to permit simultaneous synthesis of larger numbers of different polynucleotide chains. One skilled in the art will recognize, of course, that the size of the wafers may be smaller or larger than the above dimensions depending upon the specifics of the particular synthesis. Furthermore, one skilled in the art will recognize that the column dimensions will change depending on the biopolymer to be synthesized and the solid phase support used. In the production of peptides, the wafer dimensions will generally tend toward the upper limits of these ranges.

The porous materials which allow for the flow of reagents through the wafers are also formed from a chemically inert material. For example, suggested inert materials include TEFLON® and other fluorocarbon materials, such as KEVLAR®, fritted or scintered glass, and titanium and stainless steel frits. Pore size can vary, but is selected so as to allow sufficient flow of the reagents and washing solutions through the wafer, while retaining the support material and growing biopolymer chains within the wafers. A pore size of 50-100 micrometers is suggested for use with CPG supports, which are typically 120-180 micrometers in diameter. The porous material can assume a variety of designs as long as the necessary flow and containment are achieved. As illustrated and described herein, the porous material can be in the form of a flexible membrane, a rigid fritted structure, etc.

The solid phase support on which the biopolymer chain is formed can be selected from the variety of known supports. Suggested supports for polynucleotide synthesis include polystyrene-divinyl-benzene (DVB), polyamide-Kieselguhr, silica, controlled pore glass (CPG) and plastic resins such as MONOBEADS (a resin produced by Pharmacia). CPG, silica and MONOBEADS are particularly preferred as the solid phase support since they are rigid, i.e., do not swell or contract. Suggested supports for polypeptide synthesis include polystyrene and vinylbenzene resins, polyamide resins, polyamide-Kieselguhr resins, benzhydrylamine resins, and macroreticular resins.

Support materials of large pore size, for example 20-200 nm, permit good accessibility by the reagents to the growing chain and efficient washing away of reactants. Also, these supports permit assembly of relatively long chains, e.g., 50-200 residues, without steric hindrance between polymers.

The amount of the support material 22 supplied to the wafer can vary. Factors to be considered in determining the amount of support material added include the amount of DNA, RNA, polypeptide, polysaccharide or other biopolymer needed, flowrate and the extent of derivatization of the solid phase support, e.g., micromoles monomeric residue per gram of support. Advantageous results are achieved where the wafers are from two-thirds to three-fourths full, thus allowing for mixing and any possible swelling. The use of rigid solid phase supports of very large pore size, e.g., silica of 3000-4000 A, permits superior mass transfer within the supports, such that wafers can be completely filled with derivatized supports.

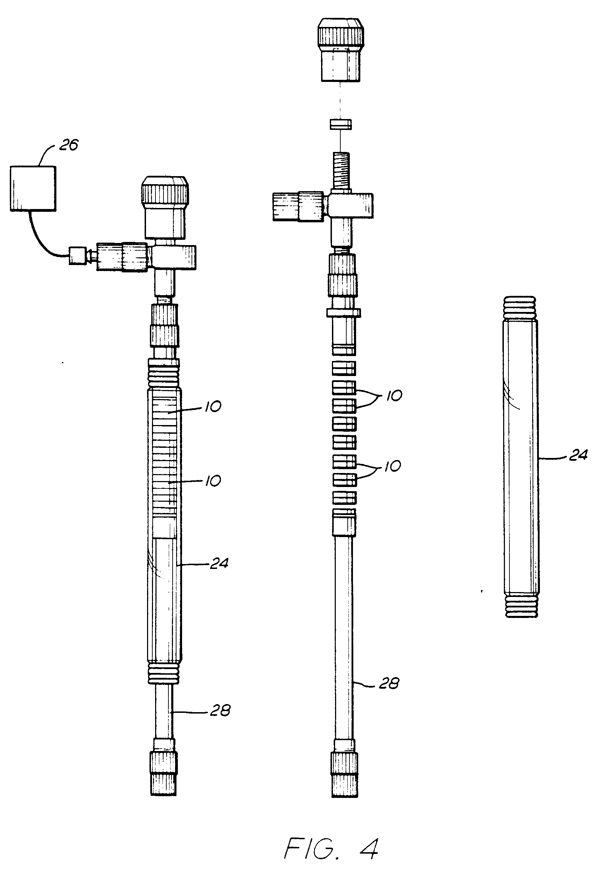

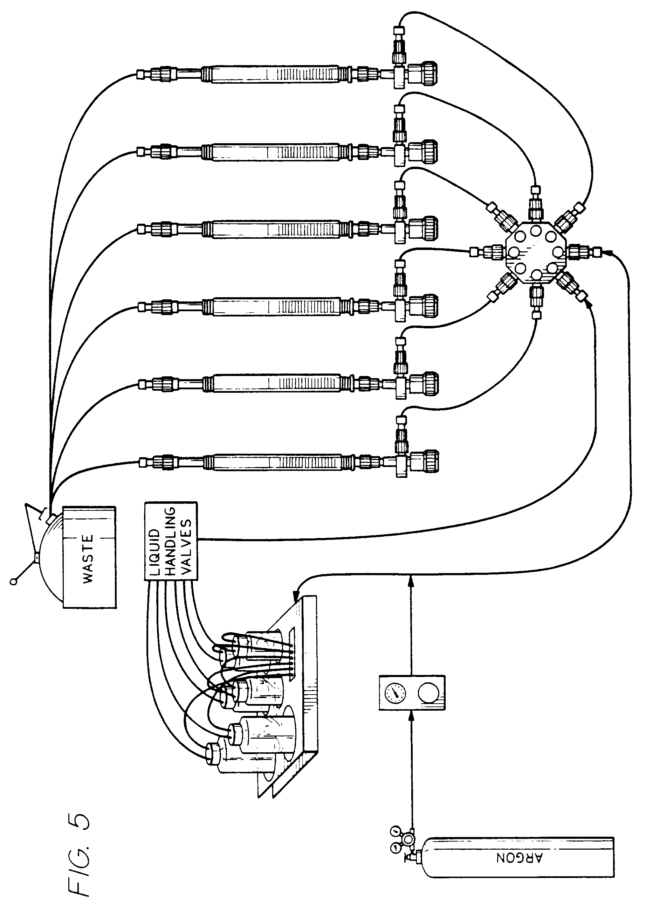

With reference to Figure 4, once assembled the wafer is placed in a column 24 through which reagents and washing solvents are passed to create a reaction cycle. The column 24 is designed to receive a number of wafers 10. As illustrated in Figures 4 and 5, delivery system 26 utilizing, for example, argon pressure, passes the reagents and washing solution through the column 24 and wafers 10. Preferably, the flow passes upwardly through the column to facilitate the reaction by causing mixing and distribution of the porous support material within a given wafer. Typically, the delivery system is connected to at least four columns in parallel corresponding to the four bases, cystosine (C), thymine (T), guanine (G) and adenine (A). Additional columns can be provided if modified bases or mixtures of bases are to be utilized in the synthesis. Any number of wafers can be placed in each column depending on the number of biopolymer sequences to be produced. For example, it is possible to place just one wafer in a column. However, this is typically costly and inefficient and, as pointed out earlier, is one problem with some of the presently available designs. Typically, the number of wafers may be in the order of 15-25 per column. However, fewer wafers or more wafers can be utilized. The number of wafers selected must, of course, allow for sufficient flow through the column. In this regard, the outer diameters of the wafers should be selected to provide a snug fit with the inner column surface to force flow through the wafers themselves, and not along the sides of the column. If additional flowrate is required, a different solvent delivery system may be utilized. Also, the number of wafers, of course, will vary with varying column heights.

The column 24 can be manufactured from any inert material. For example, glass and stainless steel are two preferred materials. The column typically includes a plunger 28, which allows for variable numbers and heights of wafers within the column.

In a further embodiment, the wafers can snap together to form, by themselves, a segmented wafer column, thereby obviating the need for a separate supporting column. In this embodiment, the delivery system 26 would be connected directly to the segmented wafer column.

In the synthesis, as previously noted, a series of columns are set up containing the wafers. Each column is provided with a reagent for residue addition. For example, in the synthesis of DNA, one column will be for the addition of cystosine (C), another for thymine (T), another for guanine (G), and another for adenine (A). Similarly for RNA synthesis, the thymine can be replaced with the reagent necessary for adding uracil (U). As previously noted, however, the number of columns used is not essential to the present invention. A single column will suffice, but this increases the time required for completion of the biopolymer synthesis. Reagents must be switched for each synthesis and fewer samples can be synthesized in one column than in four columns. Thus, use of multiple columns facilitates the number of reactions to be carried out and increases the efficiency of the procedure. A further method involves the addition of dimers, trimers, etc. In this synthesis additional columns are added. For example, with a dimer one would use 20 columns, i.e., one column for each dimer which can be added and one column for each single nucleoside base to be added.

Returning to the DNA method, wafers are selectively positioned in one of the T, G, C, or A columns, depending on the first base to be added. After the appropriate passage of the reagents and chemicals for the addition of that base to the polynucleotide chain (constituting a reaction cycle), the wafers are removed from that column, sorted for the next synthesis step, inserted into the appropriate column and the synthesis step repeated. This procedure is repeated until the desired polynucleotide sequences are synthesized. Thus, with the use of different columns for base addition, each wafer goes through its individual pattern of synthesis. This procedure allows for the concurrent synthesis of many different polynucleotides.

The present invention can be used for the production of any biopolymer by solid phase synthesis. Particularly preferred syntheses include the synthesis of polynucleotides, polypeptides and polysaccharides by solid phase methods, provided that these methods employ a flowthrough design, as implemented in the present invention. A particularly preferred solid phase route for peptide synthesis using the present invention is the previously mentioned Fmoc pentafluorophenyl ester method, utilizing polyamide-Kieselguhr supports.

The procedure is applicable to the simultaneous synthesis of multiple defined-sequence biopolymers by manual, semi-automated or fully automated procedures. For example, a semi-automated machine can be utilized that is controlled by a microcomputer. A program editor permits the operator to control the delivery of all reagents to the solid phase supports. The computer also can provide the operator, at each step, with instructions for sorting the wafers and placing them in the correct column. In the semi-automated system, the operator performs these latter functions. Of course, one skilled in the art recognizes that the fully automated system is preferred. In this system sorting of the wafers and their subsequent placement in the next column is performed by a machine.

The segmented wafer device is designed specifically for biopolymer syntheses that can be achieved by solid phase, flowthrough methods. The advantage of solid phase chemistry in the synthesis of biopolymers is that the step-wise addition to form a biopolymer is greatly facilitated because the product does not have to be purified after each condensation step. Reactants and reagents can simply be washed away. This solid phase synthetic approach has been developed for a number of different chemistries used in biopolymers synthesis. The segmented wafer can be used with all these methods.

In the synthesis of polynucleotides, the efficiency of the reaction in each step of solid phase synthesis has been measured to be between about 95 and greater than 99%, with the cycle time of approximately 5-30 minutes per nucleoside added. This approach is preferred when the quantity of desired product is in the milligram range, which is ample for most applications. In addition, solid phase synthesis is highly preferred for synthesis of mixed probe polynucleotides in which a mixture of residues exists at certain positions in the sequence.

The phosphoramidite method of solid phase synthesis (diagrammed below) is preferred for use with the present invention. 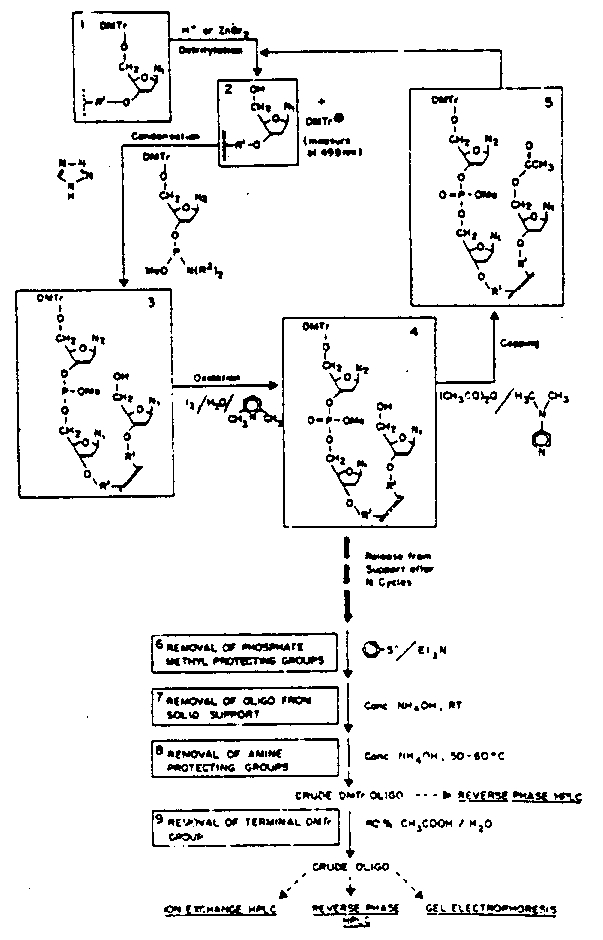
The activated intermediate in this approach is a 5'-DMT-2'-deoxynucleoside 3'-phosphoramidite. The method begins with covalent linkage of the 3'-hydroxyl group of the first nucleoside to the solid support via a long chain alkyl spacer arm.

The acid-labile dimethoxytrityl group (DMTr) is cleaved from the 5'-OH of the support-bound nucleoside by treatment with dilute aichloroacetic acid. Nucleoside phosphoramidites (at 10-20 fold molar excess over support-bound nucleoside 5'-OH) are activated by protonation of their nitrogen atom using tetrazole under anhydrous conditions, and condensation occurs as shown in step 2. At the completion of each successive coupling, the reactive phosphite is converted to a more stable phosphate using a solution of iodine in tetrahydrofuran and water (step 4). If desired, a "capping" reaction can next be carried out (with acetic anhydridedimethylaminopyridine/lutidine, step 5) to acetylate the 5'-hydroxyl groups that did not react with the activated phosphoramidite in the previous coupling, to prevent propogation of "truncated" and "nonsense" (jumbled) sequences.

At the end of each synthesis cycle, the exocyclic amino groups of A, C and G remain amide protected, the internucleotide phosphate groups are methyl esterified, and the 3'-OH end of the growing chain remains succinate-linked to the support. Prior to addition of the next residue, the detritylation step is repeated. The brilliant orange DMTr cation can be quantitated spectrophotometrically to calculate a coupling efficiency. Using the segmented wafer method, coupling efficiencies in the range of about 95-99% can be achieved.

At the end of the synthesis, the phosphate methyl protecting groups are cleaved by thiophenoxide ion, which forms from thiophenol in the presence of triethylamine (step 6). This step is not required if 2-cyanoethyl phosphoramidites are used in the synthesis. Then the alkali-labile acyl groups (protecting the exocyclic amino groups of A, G and C) and covalent linkage to the solid support are cleaved by treatment with aqueous ammonium (steps 7 and 8). If the DMTr group remains, it is cleaved by concentrated acetic acid (step 9).

As discussed previously, another method (the phosphotriester method) is commonly used for polynucleotide synthesis. Although the phosphotriester method could be adapted for use with the present invention, it is less preferred because of longer cycle times and greater requirement of anhydrous conditions which are difficult to maintain during sorting of wafers.

The previously mentioned recent developments in solid phase polynucleotide synthesis, including in situ phosphoramidite production, amidine protecting groups and ribopolymer synthesis, could be used in the present invention. Furthermore, it would be obvious to one skilled in the art to apply the present invention to future developments in biopolymer synthetic chemistry, including improvements in condensation, protection and deprotection reactions or in solid phase supports.

Use of the above synthetic methods in the segmented wafer synthesis of biopolymers can be automated using commercially available systems. For example, an automated machine containing four columns, Cruachem model PS200 synthesizer, can be controlled by an IBM PC-compatible microcomputer. The program editor permits the operator to control the delivery of all reagents to the solid phase support. This is required for the development of the reaction cycle to be used in the segmented wafer method. Furthermore, the computer can facilitate the sorting process by keeping track of the order of the wafer insertions into the columns. A computer program is utilized to direct the placement of each wafer in the appropriate columns during the synthesis. Thus, a printout indicates which wafers (identified by numbers) are to be placed into a given column after each reaction cycle, so that wafers are easily sorted and the separate wafers put in columns for the appropriate synthetic reaction sequence. The use of the computer program decreases the amount of error and increases the reliability of the synthesis. Additionally, development of an automated sorting machine, which is also controlled by the computer and interfaced with the existing synthesizer, is possible, to provide for completely automated synthesis of biopolymers using the segmented wafer device.

The present invention is further described by way of the following examples.

### EXAMPLE I

This example describes the standard procedure used for segmented synthesis of polynucleotides within chemically inert porous wafers. Details of specific applications of this procedure are given in subsequent examples.

### A. Operation of Interactive Synthesis Setup Program.

DNA sequences to be synthesized are entered (5' →3' direction) using a word processing program on an IBM compatable computer. The sequence files are stored in a non-document file and named in the format. Once the sequences are entered, the Wafer-DNA Setup Program (written in Basic) is run with the sequence files in the disk drive. The Wafer-DNA Program examines the sequence files and generates a hard copy of the following information: (i) a listing of all sequences entered, along with identifying numbers and names assigned for each, (ii) a listing of numbered wafers to be loaded with each type of derivatized CPG support (defining the 3'-terminal base in each sequence), and (iii) a schematic for directing the sorting of wafers after each reaction cycle.

### B. Reagent Preparation, Wafer Assembly and Set-up of Cruachem Model PS200 DNA Synthesizer

Using the software provided with the Cruachem DNA synthesizer, a method called "Wafer-CE20" has been created. The method is as follows:
- Method:: wafer-ce20
- Reservoir 1:: Acetonitrile
- Reservoir 2:: DMAP/THF
- Reservoir 3:: Acetic Anhydride/THF/Lutidine
- Reservoir 4:: Iodine/THF/Lutidine/Water
- Reservoir 5:: Acetonitrile
- Reservoir 6:: DCA/DCE

Method: wafer-ce20

### First Cycle

Step 1: Wash Acetonitrile Fixed
Duration = 2:15 Minutes
Step 2: Deblock DCA/DCE Base Variable

| | |
|---|---|
| A | duration = 1:30 Minutes |
| G | duration = 1:30 Minutes |
| C | duration = 2:30 Minutes |
| T | duration = 2:30 Minutes |
| Purine (A/G) | duration = 2:30 Minutes |
| Pyrimidine (T/C) | duration = 2:30 Minutes |
| N (A/C/G/T) | duration = 2:30 Minutes |

Step 3: Wash Acetonitrile Fixed
Duration = 1:30 Minutes

### Normal Cycle

Step 1: Reaction Fixed
Duration = 4:00 Minutes
Step 2: Wash Acetonitrile Fixed
Duration = 1:30 Minutes
Step 3: Wash Acetic Anhydride/THF/Lutidine Fixed
Duration = 0:12 Minutes
Step 4: Wash DMAP/THF Fixed
Duration = 0:12 Minutes
Step 5: Wash Acetic Anhydride/THF/Lutidine Fixed
Duration = 0:12 Minutes
Step 6: Wash DMAP/THF Fixed
Duration = 0:12 Minutes
Step 7: Wash Acetic Anhydride/THF/Lutidine Fixed
Duration = 0:12 Minutes
Step 8: Wash DMAP/THF Fixed
Duration = 0:12 Minutes
Step 9: Wash Acetic Anhydride/THF/Lutidine Fixed
Duration = 0:12 Minutes
Step 10: Wash Acetonitrile Fixed
Duration = 0:12 Minutes
Step 11: Cap/functionalize Fixed
Duration = 1:30 Minutes
Step 12: Wash Acetonitrile Fixed
Duration = 1:30 Minutes
Step 13: Wash Iodine/THF/Lutidine/Water Fixed
Duration = 2:00 Minutes
Step 14: Wash Acetonitrile Fixed
Duration = 1:30 Minutes
Step 15: Cap/functionalize Fixed
Duration = 60:00 Minutes
Step 16: Deblock DCA/DCE Base Variable

| | |
|---|---|
| A | duration = 1:30 Minutes |
| G | duration = 1:30 Minutes |
| C | duration = 2:30 Minutes |
| T | duration = 2:30 Minutes |
| Purine (A/G) | duration = 1:30 Minutes |
| Pyrimidine (T/C) | duration = 2:30 Minutes |
| N (A/C/G/T) | duration = 2:30 Minutes |

Step 17: Wash Acetonitrile Fixed
Duration = 1:30 Minutes

### Final Cycle

Step 1: Reaction Fixed
   Duration = 4:00 Minutes
Step 2: Wash Acetonitrile Fixed
   Duration = 1:30 Minutes
Step 3: Wash Iodine/THF/Lutidine/Water Fixed
   Duration = 2:00 Minutes
Step 4: Wash Acetonitrile Fixed
   Duration = 4:00 Minutes

Before synthesis begins it is necessary to assemble the wafers. For each wafer, the bottom portion of the wafer is assembled first so that the derivatized controlled pore glass (CPG) support material can be added through the top. Approximately 18 mg of the appropriate CPG is added, as directed by the printout from the Wafer-DNA Setup Program. Finally the wafer is closed by placing another piece of the porous TEFLON® cloth over the reaction chamber and securely fastening this with the outer TEFLON® retaining ring. the wafers are loaded into the appropriate columns, as directed in step 2 of the printout from the Wafer-DNA Setup Program.

To prepare the synthesizer for operation, the reservoirs are filled with their respective reagents and the solvent lines are flushed, using the operating program supplied with the Cruachem synthesizer. The last reagents to be prepared are the phosphoramidites and the sublimed tetrazole. Table I describes the reagents used for polynucleotide synthesis by the segmented wafer method.
NORMAL CYCLE: Wafer - CE20 Method
SOLVENTS/REAGENTS PER COLUMN OF 10 WAFERS
1. Acetonitrile - 12.5ml (with solvent organizer stand, use 11 reservoir)
2. 6.5% Dimethylaminopyridine in THF (w/v) - 1.2ml
3. Acetic anhydride/THF/Lutidine - 1.6ml
4. Iodine (0.1M in water/lutidine/THF - 1:10:4) - 4ml
5. 3% Dichloroacetic acid/dichloroethane (w/v) - 4ml
SOLVENT FLOW RATE: 2ml/min
AVERAGE CYCLE TIME: 18 min
SYNTHESIS SCALE: 0.5-1 micromole per wafer
SUPPORT: Nucleoside-CPG, typically 15-20 mg per wafer
MONOMER SOLUTION: 0.1M CE phosphoramidite
6.67 ml acetonitrile/.5 g T-phosphoramidite
6.00 ml acetonitrile/0.5 g G-phosphoramidite
5.80 ml acetonitrile/0.5 g A-phosphoramidite
6.00 ml acetonitrile/0.5 g C-phosphoramidite
Catalyst - 0.5M tetrazole (20 ml acetonitrile/0.7 g tetrazole)
Mix 0.5ml monomer and 0.5ml catalyst and inject into the column.

### C. Synthesis of Polynucleotides

Using the PS200 Cruachem DNA Synthesizer and resident operating software, the Wafer-CE20 method and the segmented wafer synthesis device depicted in Figure 5, the segmented synthesis of polynucleotides is carried out, employing the previously described 2-cyanoethyl phosphoramidite chemistry. After the wafer-containing columns are connected to the synthesizer, the first cycle, consisting only of detritylation and washing, is carried out as indicted in the method ("First Cycle," Steps 1 through 3). Initiation of each subsequent cycle occurs upon injection of phosphoramidites, immediately preceding Step 1 ("Normal Cycle"). Step 15 ("Normal Cycle") is not a repeat of capping Step 11, but rather is a variable "pause" period during which the wafers are sorted, as directed by the printout from the Wafer-DNA Setup Program. In each normal cycle, as soon as sorting of wafers is completed and columns are reconnected to the synthesizer, the synthesis cycle is resumed and detritylation and washing are carried out. The final cycle is identical to the normal cycle, except that capping and detritylation are omitted. If desired, after synthesis within all wafers is complete, the wafers can be reassembled into columns and subjected to detritylation to remove the remaining 5'-DMT protecting groups.

After the synthesis has been completed, the wafer contents are emptied into screw-top vials and the DNA is cleaved from the support, further deblocked and purified by prior-art procedures, following the instructions provided in the Cruachem PS200 operation manual.

The above procedure has been carried out numerous times, resulting in the simultaneous synthesis (at a scale of 0.5-1.0 micromole) of between 3 and 79 different DNA sequences in a single day, of length ranging from 15 to 25 residues. The coupling efficiency at each step was typically about 95% and DNA sequences have been confirmed by the Maxam-Gilbert sequencing method.

### EXAMPLE II

### Simultaneous Synthesis of Three Test Polynucleotides

To assess the usefulness of the segmented wafer synthesis device for biopolymer synthesis, simultaneous synthesis of three pentadecamers was carried out, using the equipment illustrated in Figures 1-5 and the general procedure described in Example 1, as further detailed below. The nucleotide sequences of the test DNA molecules were:
1. 5'-GAGCCATCAAGCCAG-3'
2. 5'-GCTGCAGAGAGGCCA-3'
3. 5'-GAGGTGTTGGAGCTG-3'
The details of the synthesis are:
SOURCE OF REAGENTS: Cruachem
SCALE OF SYNTHESIS AND WAFER DIMENSIONS: Each wafer (10 mm o.d. x 4 mm h.) contained 18 mg of nucleoside-CPG (approximately 0.6 micromole) and was assembled from components of the following dimensions (see Figures 1-3): Porous Teflon® cloth, 12 mm diameter; Inner housing ring, 4 mm i.d. x 4 mm h.; Outer retaining rings, 10 mm o.d. x 2 mm h.; Internal volume, 0.050 ml.

REACTION CYCLE AND REAGENT/SOLVENT USAGE: The standard "CE Phosphoramidite" protocol and reaction cycle, as specified in the Cruachem PS200 Synthesizer instruction manual for prior-art operation, was used in this experiment. The "standing" steps of the reaction cycle (condensation step 1 and capping step 11) were carried out for the same times given in Example I for the "wafer-CE20" method (4.0 and 1.5 minutes, respectively). Step 1 was initiated by mixing 0.1 ml of 0.1M CE phosphoramidite and 0.1 ml of 0.5M tetrazole (both in anhydrous acetonitrile) in a syringe and injecting the mixture into each column. The remaining "flow" steps in the reaction cycle were carried out (at 2 ml/min) for one-half the time specified in Example I for the "normal cycle" of the "wafer-CE20" method. The columns were briefly flushed with Argon just prior to the sorting step 15. The average cycle time was 11 minutes. The quantity of reagents consumed per cycle per wafer, along with approximate cost per base addition (based on catalog price of nucleoside-CPG, reagents and solvents) were as follows:
3.7 ml acetonitrile
0.4 ml 6.5% dimethylaminopyridine in THF
0.5 ml acetic anhydride/THF/Lutidine
1.2 ml iodine (0.1M in water/lutidine/THF-1:10:4)
1.2 ml 6.3% dichloroacetic acid/dichloroethane
0.06 ml 0.5M tetrazole in aetonitrile
0.06 ml 0.M 2-cyanoethyl phosphoramitite in acetonitrile
Cost per base addition: $1.98, compared with $5.42/base if synthesis were carried out by the Cruachem PS200 Synthesizer, operated in the standard (prior-art) mode.

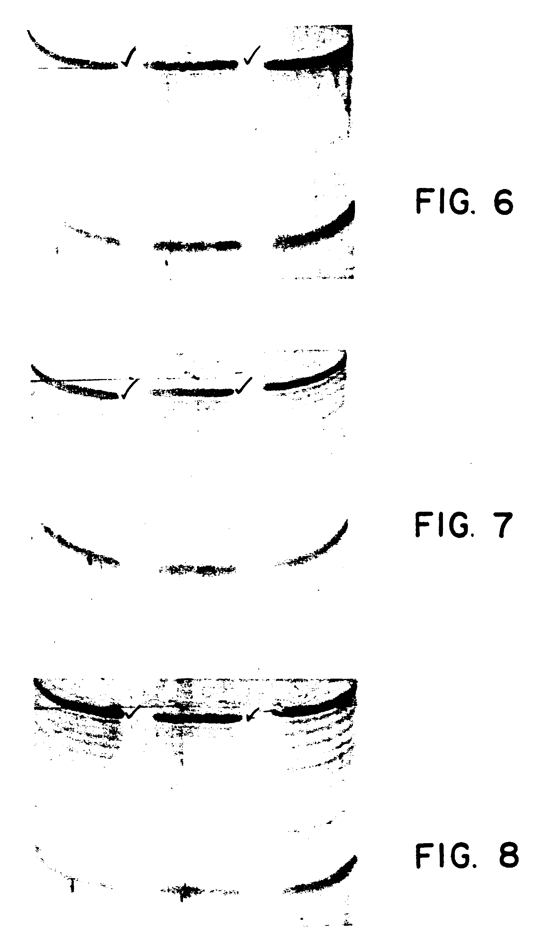

POST-SYNTHESIS IS DEPROTECTION, DNA PURIFICATION, ANALYSIS: The final detritylation step was carried out on the column (as in step 16 of the "normal cycle"). After wafer contents were emptied into 1.5 ml eppendorf tubes, 1 ml of fresh concentrated ammonium hydroxide was added, tubes were capped and mixed. After 20 minutes at room temperature (during which cleavage of polynucleotides from the CPG occurred), the liquid, along with 1 ml additional concentrated ammonium hydroxide, was transferred to a screw-top glass vial (15 ml o.d. x 45 mm height), tightly sealed with a Teflon®-lined cap, and incubated at 55 degrees C for 6-15 hours (to deprotect exocyclic amino groups of C, A and G). The ammonia was removed by vacuum, using a Savant SpeedVac concentrator (1 hr by water jet, followed by overnight at high vacuum). The dried DNA was dissolved in a small volume of water, then purified by electrophoresis (20% polyacrylamide, 7M urea), visualized by "UV-shadow" gels produced from 20 A₂₆₀ units of the crude reaction products are represented by Figure 6.

The uppermost band in each gel represents the desired full-length product, the faint lower bands represent "failure" sequences, and the dark band at the bottom represents the bromophenol blue marker dye. These gels were comparable to those obtained with similar DNA products produced on an automated Applied Biosystems Model 380A Synthesizer (using prior-art phosphoramidite procedure) and during which coupling efficiencies were measured (by the standard trityl release assay) to be 98-99%. Thus, the average coupling efficiency in the synthesis of the three pentadecamers by the segmented wafer synthesis device was estimated to be about 98-99%.

COMMENTS: These DNA products were successfully 5'-phosphorylated (using T4 polynucleotide kinase) and used as hybridization probes, by prior-art procedures. The high yield and quality and reduced cost of the products demonstrates the usefulness of the present invention for simultaneous polynucleotide synthesis. Furthermore, an important finding is that the manual sorting process carried out after each reaction cycle does not negatively affect the synthesis.

### EXAMPLE III

### Simultaneous Synthesis of 62 Biopolymer

To assess the utility of the present invention for simultaneous synthesis of large numbers of biopolymers, 62 different DNA nonadecamers were synthesized, using the equipment illustrated in Figures 1-5 and the general procedure outlined in Example I, as further detailed below. The nucleotide sequences of the test DNA molecules were:
1. 5'-GAAAGGTTAGATTCCTCAC-3'
2. 5'-AAGAAAGGTCAAATTCCTC-3'
3. 5'-TGGTGGAAGCAAGGTTAAA-3'
4. 5'-GCTTGGTGGCAGAAAGGTT-3'
5. 5'-GAATGGTTTCTAACTGCTT-3'
6. 5'-TTTTCAAAGCGAATGGTTT-3'
7. 5'-GGTTTAATGTCCTGTTTTT-3'
8. 5'-GGCGTTTTCATCAGCGGTT-3'
9. 5'-CCACCCGGCCTTTTCTTCA-3'
10. 5'-GACGGCGCGTCACCCGGCC-3'
11. 5'-GTTGACGGCTCGCCACCCG-3'
12. 5'-TCTTCCAGTTCCTCTTCCG-3'
13. 5'-AGTTCTTCCCGTACCTCTT-3'
14. 5'-CCAGACCACCATACTCCAG-3'
15. 5'-GATTTCAGCATCGCCAGAC-3'
16. 5'-AGCGGCAGCATGTCGGTGT-3'
17. 5'-TGCACACGCTCGGTTTTCG-3'
18. 5'-TATGCACACCCCCGGTTTT-3'
19. 5'-AAGAGGTATCCACACGCCC-3'
20. 5'-GGTGATAAGCGGTATGCAC-3'
21. 5'-CAACGTCCCCTTGCAGTTA-3'
22. 5'-ATAAACGTCTCGTTGCAGT-3'
23. 5'-ACGATAAACCTCCCGTTGC-3'
24. 5'-TTTGCAGGTCAGGATCGGT-3'
25. 5'-ATCTTTTGCCGGTTAGGAT-3'
26. 5'-CGCACCGGACTGTTTTGCA-3'
27. 5'-TCGTTACGCTCCGGAATGT-3'
28. 5'-CTTCGTTACCCACCGGAAT-3'
29. 5'-TACGACGACGTTCTTCGTT-3'
30. 5'-ACGCCTGGCCGATACGACG-3'
31. 5'-GCAATAAACTCCTGGCGGA-3'
32. 5'-GGCGCAATAGACGCCTGGC-3'
33. 5'-TCCTCTGGCTCAATAAACG-3'
34. 5'-CAATCTGCGCGTAGtCCGC-3'
35. 5'-ATAATGCGCCGTTCAATCT-3'
36. 5'-CCATAATGCCCAGTTCAAT-3'
37. 5'-GAAAGATGCTCCATAATGC-3'
38. 5'-GAAAGATGCTCCATAATGC-3'
39. 5'-GCGAAAGATCCGCCATAAT-3'
40. 5'-CGCTACGGCCTTGCTCGCT-3'
41. 5'-ATCGCTTTCTCGCTACGGC-3'
42. 5'-TGATCGCTTCCGCGCTACG-3'
43. 5'-AAATCAGACGAAAGTTGAT-3'
44. 5'-TCATGCCATCAATCAGACC-3'
45. 5'-CACTCATGCGATAAATCAG-3'
46. 5'-GAGCACGGGCGCGTTCCAT-3'
47. 5'-TTCGCCTGATCACGGGTGC-3'
48. 5'-TGCTCTTTCTCCTGAGCAC-3'
49. 5'-CGTCCGTCCCGCGTTTCAA-3'
50. 5'-GACGGCGTCTGTCCAGCGT-3'
51. 5'-ACAGACGGCCTCCGTCCAG-3'
52. 5'-GATACAGACCGCGTCCGTC-3'
53. 5'-TTAATGGCTTCACGTTCAG-3'
54. 5'-GCGTTAATGTCTGCACGTT-3'
55. 5'-TTGGCGCGTCAATGGCTGC-3'
56. 5'-CGGTTCCCTCCATTGGCGC-3'
57. 5'-ATGTCGGCGTCGGTTCCCT-3'
58. 5'-CGTTTGATACTGTCGGCGG-3'
59. 5'-TCGCCCGTTCGATAATGTC-3'
60. 5'-TCAACGGCACTCATCGCCC-3'
61. 5'-TCATCGTGTTCCTGCATGA-3'
62. 5'-GTTCATCGTCTACCTGCAT-3'
The details of the synthesis are:
SOURCE OF REAGENTS: Cruachem
SCALE OF SYNTHESIS AND WAFER DIMENSIONS: As in Example II, 18 mg of derivatized CPG (approximately 0.6 micromole) was placed into each wafer of dimensions, 10 mm o.d. x 4 mm height.

REACTION CYCLE: The synthesis was carried out using the "wafer-ce20" method (reaction cycle as listed in Example 1). A mixture of 0.5 ml 0.5M tetrazole and 0.5ml 0.1M 2-cyanoethyl phosphoramidite was injected upwards through the column of wafers to initiate step 1. Duration of average reaction cycle was 18 minutes. Average time required for sorting (step 15) was 12 minutes.

REAGENT AND SOLVENT USAGE PER REACTION CYCLE: The quantities of reagents and solvents required per base addition per wafer, and cost of synthesis per base addition were:
0.80 ml acetonitrile
0.08 ml 6.5% dimethylaminopyridine in THF
0.10 ml acetic anhydride/THF/Lutidine
0.26 ml iodine (0.1M in water/lutidine/THF-1:10:4)
0.26 ml 6.3% dichloroacetic acid/dichloreothane
0.03 ml 0.5M tetrazole in acetonitrile
0.03 ml 0.1M 2-cyanoethyl phosphoramidite in acetonitrile
Cost per base addition: $0.65. This value is only about 1/8 the cost of synthesis that would pertain to synthesis of these same polynucleotides by the Cruachem PS200 Synthesizer, or by the fully automated Applied Biosystems Model 380A, operated in the standard (prior-art) mode.

TOTAL TIME REQUIRED FOR SYNTHESIS OF 62 POLYNUCLEOTIDES: Synthesis was completed in a single day, over a period of 12 hours. This compares with approximately ten days required to produce this number of polynucleotides using a 3-column, fully automated Applied Biosystems Model 380A Synthesizer, operating at two syntheses per column per day.

POST-SYNTHESIS DEPROTECTION, DNA PURIFICATION, ANALYSIS: Procedures were the same as those given in Example II. The "UV shadowing" gels illustrated in Figures 7 and 8, are representative of those obtained with 20 A₂₆₀ units of crude reaction products formed in this experiment.

Based on the results of UV shadowing gel analyses and quantitation of purified DNA products, the average coupling efficiency during this multiple simultaneous synthesis was estimated to be 92-98%.

COMMENTS: The purified polynucleotides were used in prior-art procedures for oligonucleotide-directed mutagenesis. During this work the DNA products were successfully 5'-phosphorylated (using T4 polynucleotide kinase), annealed to the DNA templates, and elongated by DNA polymerase. Thus, DNA products of high quality were produced, at high yields and at greatly reduced cost and requiring greatly reduced time, compared to prior-art procedures.

Thus, the wafers of the present invention provide for the synthesis of multiple defined-sequenced biopolymers. The geometry of the support material results in high coupling efficiencies, and the rigid wafers facilitate sorting after each reaction cycle. Extremely advantageous are the reduced synthesis cost realized by the present invention and the decreased time required for synthesis of large numbers of biopolymers. The economic and time-saving advantages created by the segmented wafer method should increase demand for the commercial product and fuel future developments in biomedical science.

The present invention, therefore, is well adapted to carry out the objects and attain the ends and the advantages mentioned as well as those inherent therein. While presently preferred embodiments of the invention have been given for the purpose of disclosure, numerous changes in the details of construction and arrangement of parts can be made which will readily suggest themselves to those skilled in the art and which are encompassed within the scope of the appended claims.

## Claims

1. A modular chemically inert wafer for synthesizing biopolymers adapted to be abuttably stacked in a multiple relationship wherein each said wafer has independent upper and lower porous means when in a stacked relationship, comprising:
a solid phase support material capable of covalently binding a biopolymer residue;
a housing ring having inner and outer walls, for retaining said support material in a chamber formed by the inner walls and coaxially extending therethrough said housing ring; and
upper and lower porous means positioned at each end of said housing ring wherein said porous means allow flow through said housing ring to said support material and prevent migration of said support material from said housing ring;

2. A wafer as claimed in claim 1, wherein said housing ring comprises an inner, enclosed reaction chamber for receiving and retaining said support material, said housing ring being open on both ends.

3. A wafer as claimed in claim 2, wherein said porous flow means comprises separate means provided at each end of said housing ring and extending across said open ends to enclose said chamber.

4. A wafer as claimed in claim 1, further comprising securing means for securing said porous flow means to said housing ring.

5. A wafer as claimed in claim 4, wherein said securing means is chemically inert.

6. A wafer as claimed in claim 1, wherein said housing ring is chemically inert.

7. A wafer as claimed in claim 1, wherein said solid phase support material is silica, controlled pore glass, polystyrene-divinyl-benzene, polyamide-Kieselguhr, benzyl-linked polystyrene resins, spacer-linked styrene resins, polyamide resins or macroreticular resins.

8. A wafer as claimed in claim 7, wherein said solid phase support material comprises controlled pore glass.

9. A wafer as claimed in claim 1, wherein said solid phase support material comprises a derivatized material which includes a covalently attached residue.

10. A wafer as claimed in claim 1 wherein said porous flow means comprises a fluorocarbon material, fritted or sintered glass, titanium or stainless steel frits.

11. A wafer as claimed in claim 1, wherein the porosity of said flow means is sufficiently large to allow flow through the wafer and sufficiently small to retain said solid phase support material in the wafer.

12. A wafer as claimed in claim 3, wherein said porous flow means are pressure fitted in said housing ring.

13. A modular chemically inert wafer for synthesizing biopolymers adapted to be abuttably stacked in a multiple relationship wherein each said wafer has independent upper and lower porous means when in a stacked relationship, comprising:
a solid phase support material capable of covalently binding a biopolymer residue;
an inner housing ring having inner and outer walls comprising an inner reaction chamber formed by the inner walls of said ring and coaxially extending therethrough for receiving and retaining said support material, said housing ring being open at both ends;
an inert porous membrane positioned at and extending across each of said open ends of said inner housing ring, said membrane having a diameter larger than the outermost diameter of said housing ring so that said membrane extends beyond said outer wall; and
two outer sleeve-like rings having an inner diameter slightly larger than said housing ring and the thickness of said membrane for securing the edges of said membrane between said housing ring and said outer sleeve-like rings.

14. A segmented wafer synthesis device for the synthesis of multiple defined-sequence biopolymers, comprising:
a solvent delivery system;
at least one column connected to said solvent delivery system to provide solvent and reagent flow through said column; and
at least one wafer as claimed in claim 1 positioned in said column at which polymeric synthesis occurs.

15. A synthesis device as claimed in claim 14, further comprising at least four columns for receiving four reagents, and a plurality of wafers in each column, wherein each of said wafers provides for the synthesis of a defined-sequence biopolymer.

16. A segmented wafer synthesis device for the synthesis of multiple defined-sequence biopolymers, comprising:
a solvent delivery system; and
a plurality of stacked wafers as claimed in claim 1, each of said wafers including a biopolymeric synthesis material and being connected to the next adjacent wafer to form a column, wherein each said wafer directly abuts the adjacent wafer and wherein said solvent delivery system is connected to said column to provide flow through said column.

## Patentansprüche

1. Modulare, chemisch inerte, zum aneinander anliegenden Stapeln in Mehrfachanordnung ausgestattete Scheibe zur Synthese von Biopolymeren, wobei in gestapelten Anordnung unabhängige obere und untere porösen Mittel aufweist, umfassend
ein Festphasenträgermaterial mit der Fähigkeit zum kovalenten Binden eines Biopolymerrests,
einen Gehäusering mit innerer und äußerer Wand zum Festhalten des Trägermaterials in einer von den Innenwänden gebildeten und sich koaxial durch den Gehäusering hindurch erstreckenden Kammer und
an jedem Ende des Gehäuserings angeordnete obere und untere poröse Mittel, die einen Fluß durch den Gehäusering zu dem Trägermaterial gewährleisten und eine Wanderung des Trägermaterials aus dem Gehäusering verhindern.

2. Scheibe nach Anspruch 1, wobei der an beiden Enden offene Gehäusering eine innere, umschlossene Reaktionskammer zur Aufnahme und zum Festhalten des Trägermaterials umfaßt.

3. Scheibe nach Anspruch 2, wobei die poröse Fließvorrichtung an jedem Ende des Gehäuserings vorgesehene und sich durch die offenen Enden erstreckende getrennte Einrichtungen zum Umschließen der Kammer umfaßt.

4. Scheibe nach Anspruch 1, des weiteren umfassend eine Befestigungsvorrichtung zum Befestigen der porösen Fließvorrichtung an dem Gehäusering.

5. Scheibe nach Anspruch 4, wobei die Befestigungsvorrichtung chemisch inert ist.

6. Scheibe nach Anspruch 1, wobei der Gehäusering chemisch inert ist.

7. Scheibe nach Anspruch 1, wobei das Festphasenträgermaterial aus Siliziumdioxid, einem gesteuerte Poren aufweisenden Glas, Polystyroldivinylbenzol, Polyamidkieselgur, benzylverknüpften Polystyrolharzen, durch eine Abstandsgruppe verknüpften Styrolharzen, Polyamidharzen oder eine Makronetzstruktur aufweisenden Harzen besteht.

8. Scheibe nach Anspruch 7, wobei das Festphasenträgermaterial gesteuerte Poren aufweisendes Glas umfaßt.

9. Scheibe nach Anspruch 1, wobei das Festphasenträgermaterial ein einen kovalent gebundenen Rest umfassendes abgeleitetes Material umfaßt.

10. Scheibe nach Anspruch 1, wobei die poröse Fließvorrichtung ein Fluorkohlenstoffmaterial, Fritte- oder Sinterglas, Titan- oder nichtrostende Stahlfritten umfaßt.

11. Scheibe nach Anspruch 1, wobei die Porosität der Fließvorrichtung zur Gewährleistung eines Fließens durch die Scheibe ausreichend groß und zum Festhalten des Festphasenträgermaterials in der Scheibe ausreichend klein ist.

12. Scheibe nach Anspruch 3, wobei die poröse Fließvorrichtung in den Gehäusering durch Druck eingepaßt ist.

13. Modulare, chemisch inerte, zum aneinanderliegenden Stapeln in Mehrfachanordnung ausgestaltete Scheibe zur Synthese von Biopolymeren, wobei jede Scheibe bei vorliegen in einer gestapelten Anordnung unabhängige obere und untere poröse Mittel aufweist, umfassend ein Festphasenträgermaterial mit der Fähigkeit zum kovalenten Binden eines Biopolymerrests,
einen inneren Gehäusering mit Innen- und Außenwänden mit einer durch die Innenwände des Rings gebildeten und sich koaxial durch diesen hindurch erstreckenden inneren Reaktionskammer zur Aufnahme und zum Festhalten des Trägermaterials, wobei der Gehäusering an beiden Enden offen ist,
eine an jedem der offenen Enden des inneren Gehäuserings angeordnete und sich durch diesen hindurch erstreckende inerte poröse Membran eines über dem Außendurchmesser des Gehäuserings liegenden Durchmessers, so daß sich die Membran über die Außenwand hinaus erstreckt, und
zwei äußere hülsenartige Ringe mit einem Innendurchmesser, der etwas über dem des Gehäuserings und der Dicke der Membran liegt, zum Befestigen der Kanten der Membran zwischen dem Gehäusering und den äußeren hülsenartigen Ringen.

14. Unterteilte Scheibensynthesevorrichtung zur Synthese von mehreren sequenzdefinierten Biopolymeren, umfassend ein Lösungsmittelabgabesysten,
mindestens eine mit dem Lösungsmittelabgabesystem verbundene Säule zur Gewährleistung eines Lösungsmittel- und Reagenzflusses durch die Säule und
mindestens eine Scheibe nach Anspruch 1, die in der Säule, in der die Polymersynthese abläuft, angeordnet ist.

15. Synthesevorrichtung nach Anspruch 14, des weiteren umfassend mindestens vier Säulen zur Aufnahme von vier Reagenzien und eine Mehrzahl von Scheiben in jeder Säule, wobei jede der Scheiben eine Synthese eines eine definierte Sequenz aufweisenden Biopolymeren gewährleistet.

16. Unterteilte Scheibensynthesevorrichtung zur Synthese von mehreren sequenzdefinierten Biopolymeren, umfassend ein Lösungsmittelabgabesystem und
eine Mehrzahl von gestapelten Scheiben nach Anspruch 1, wobei jede Scheibe ein Biopolymersynthesematerial umfaßt und mit der benachbarten Scheibe unter Bildung einer Säule verbunden ist, wobei jede Scheibe direkt auf der benachbarten Scheibe aufliegt und wobei das Lösungsmittelabgabesystem mit der Säule zur Gewährleistung eines Flusses durch die Säule verbunden ist.

## Revendications

1. Segment en forme de tranche ("wafer"), modulaire, chimiquement inerte, pour la synthèse de biopolymères, dont plusieurs exemplaires peuvent être empilés bout à bout et mis ainsi en relation, chacun desdits segments présentant des moyens poreux supérieurs et des moyens poreux inférieurs, indépendants, lorsqu'il est dans un empilement, ledit segment comprenant :
un matériau support constituant une phase solide, capable de fixer de manière covalente un reste de biopolymère,
un anneau de logement, présentant des parois internes et des parois externes, pour retenir ledit matériau support dans une chambre formée par les parois internes et s'étendant coaxialement à travers ledit anneau de logement, et
des moyens poreux supérieurs et des moyens poreux inférieurs, placés à chaque extrémité dudit anneau de logement, lesdits moyens poreux permettant l'écoulement à travers ledit anneau de logement, vers ledit matériau support, et empêchant la migration dudit matériau support hors dudit anneau de logement.

2. Segment selon la revendication 1, dans lequel ledit anneau de logement comprend une chambre de réaction interne, enfermée, destinée à recevoir et à retenir ledit matériau support, ledit anneau de logement étant ouvert aux deux extrémités.

3. Segment selon la revendication 2, dans lequel lesdits moyens poreux pour l'écoulement comprennent des moyens séparés, présents à chaque extrémité dudit anneau de logement et s'étendant sur lesdites extrémités ouvertes pour clore ladite chambre.

4. Segment selon la revendication 1, comprenant en outre des moyens de fixation pour la fixation desdits moyens poreux pour l'écoulement audit anneau de logement.

5. Segment selon la revendication 4, dans lequel lesdits moyens de fixation sont chimiquement inertes.

6. Segment selon la revendication 1, dans lequel ledit anneau de logement est chimiquement inerte.

7. Segment selon la revendication 1, dans lequel ledit matériau support constituant une phase solide est de la silice, du verre de porosité réglée, du polystyrène-divinylbenzène, du polyamide-kieselguhr, une résine de polystyrène comportant des liaisons benzyle, une résine de styrène comportant des liaisons par un fragment espaçant, une résine de polyamide ou une résine macroréticulaire.

8. Segment selon la revendication 7, dans lequel ledit matériau support constituant une phase solide comprend du verre de porosité réglée.

9. Segment selon la revendication 1, dans lequel ledit matériau support constituant une phase solide comprend un matériau transformé en un dérivé, qui renferme un reste lié de manière covalente.

10. Segment selon la revendication 1, dans lequel lesdits moyens poreux pour l'écoulement comprennent un matériau fluorocarboné, du verre fritté ou aggloméré par frittage, du titane fritté ou de l'acier inoxydable fritté.

11. Segment selon la revendication 1, dans lequel la taille de pore desdits moyens pour l'écoulement est suffisamment grande pour permettre l'écoulement à travers le segment et suffisamment petite pour retenir ledit matériau support constituant une phase solide dans le segment.

12. Segment selon la revendication 3, dans lequel lesdits moyens poreux pour l'écoulement sont montés par pression dans ledit anneau de logement.

13. Segment en forme de tranche, modulaire, chimiquement inerte, pour la synthèse de biopolymères, dont plusieurs exemplaires peuvent être empilés bout à bout et mis ainsi en relation, chacun desdits segments présentant des moyens poreux supérieurs et des moyens poreux inférieurs, indépendants, lorsqu'il est dans un empilement, ledit segment comprenant :
un matériau support constituant une phase solide, capable de fixer de manière covalente un reste de biopolymère,
un anneau de logement interne, présentant des parois internes et des parois externes, comprenant une chambre de réaction interne, formée par les parois internes dudit anneau et s'étendant coaxialement d'un bout à l'autre de l'anneau, destinée à recevoir et à retenir ledit matériau support, ledit anneau de logement étant ouvert aux deux extrémités,
une membrane poreuse inerte, placée à et s'étendant sur chacune desdites extrémités ouvertes dudit anneau de logement interne, ladite membrane ayant un diamètre supérieur au diamètre extérieur dudit anneau de logement, de sorte que ladite membrane s'étend au-delà de ladite paroi externe, et
deux anneaux externes, en forme de manchon, présentant un diamètre interne légèrement supérieur audit anneau de logement et à l'épaisseur de ladite membrane, pour la fixation des bords de ladite membrane entre ledit anneau de logement et lesdits anneaux externes, en forme de manchon.

14. Dispositif de synthèse segmenté, à segment en forme de tranche, pour la synthèse de biopolymères multiples, de séquence définie, comprenant :
un système d'apport de solvant,
au moins une colonne reliée audit système d'apport de solvant, qui permet l'écoulement de solvant et de réactif à travers ladite colonne, et
au moins un segment en forme de tranche, tel que revendiqué dans la revendication 1, placé dans ladite colonne, où s'effectue la synthèse du polymère.

15. Dispositif de synthèse selon la revendication 14, comprenant au moins quatre colonnes destinées à recevoir quatre réactifs, et plusieurs segments dans chaque colonne, chacun desdits segments accomplissant la synthèse d'un biopolymère de séquence définie.

16. Dispositif de synthèse segmenté, à segment en forme de tranche, pour la synthèse de biopolymères multiples de séquence définie, comprenant :
un système d'apport de solvant, et
plusieurs segments en forme de tranche, tels que revendiqués dans la revendication 1, empilés, chacun desdits segments comprenant un matériau de synthèse de biopolymère et étant relié au segment suivant adjacent pour former une colonne, chacun desdits segments étant mis directement bout à bout avec le segment adjacent et ledit système d'apport de solvant étant relié à ladite colonne pour permettre l'écoulement à travers ladite colonne.
